# EUROPÄISCHE PATENTSCHRIFT

(11) **EP 3 213 726 B1**
(45) Veröffentlichungstag und Bekanntmachung des Hinweises auf die Patenterteilung: **01.01.2020**
(21) Anmeldenummer: 16158451.1
(22) Anmeldetag: 03.03.2016
(51) Int. Cl.: A61F 9/06

(54) **SCHUTZVORRICHTUNG**
PROTECTION DEVICE
DISPOSITIF DE PROTECTION

(43) Veröffentlichungstag der Anmeldung: 06.09.2017
(73) Patentinhaber: Optrel Holding AG, 9630 Wattwil (CH)
(72) Erfinder: HOFER-KRANER, Ramon, 9100 Herisau (CH); HEUSSER, Jonathan, 8713 Uerikon (CH); BLÖCHLINGER, Daniel, 8735 St. Gallenkappel (CH)
(74) Vertreter: Daub, Thomas

(56) Entgegenhaltungen:
- EP-A1- 2 839 817
- EP-A1- 3 000 447
- DE-A1-102006 048 204
- US-A1- 2008 060 102
- US-A1- 2011 179 541

## Beschreibung

### Stand der Technik

Die Erfindung betrifft eine Schutzvorrichtung, insbesondere eine Blendschutzvorrichtung.

Aus der US 2011/0179541 A1 ist bereits eine Linsenanordnung zum Befestigen in einer Öffnung in einer Frontplatte eines Gesichtsschutzes bekannt, welche eine erste Halterlinse mit Form und Abmessungen aufweist, die zur Befestigung in der vorderen Öffnung einer Frontplatte konfiguriert ist und eine Krümmung aufweist, die im Wesentlichen der Krümmung der Frontplatte entspricht. Eine zweite Linse mit kleineren Abmessungen ist hinter der ersten Linse befestigt. Die Linsenanordnung kann lösbar in der Frontplattenöffnung befestigt sein, und die zweite Linse ist mit Sätzen von Linsen mit unterschiedlichen Eigenschaften austauschbar. Die Halterungsbaugruppe hat eine hintere Abdeckung und eine Dichtung oder einen Abstandshalter zwischen der hinteren Abdeckung und der zweiten Linse, um zu ermöglichen, dass zwei Linsen unterschiedlicher Tiefe in der Anordnung gesichert werden.

Aus der EP 2 839 817 A1 ist bereits eine Schutzvorrichtung bekannt, mit zumindest einem optischen Blendschutzfilter, welcher einen Nasenausschnitt aufweist, mit zumindest einer Sensoreinheit, welche zu einer Erfassung eines Arbeitszustands und einer davon abhängigen Ansteuerung einer Durchlässigkeit des Blendschutzfilters vorgesehen ist, mit zumindest einer Schildeinheit, in welcher der zumindest eine Blendschutzfilter fest aufgenommen ist, und mit zumindest einer Kopfbefestigungseinheit zu einer Befestigung an einem Kopf eines Benutzers.

Die Aufgabe der Erfindung besteht insbesondere darin, eine gattungsgemäße Vorrichtung mit verbesserten Eigenschaften hinsichtlich eines Benutzerkomforts, sowie hinsichtlich einer Sicherheit bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von Schutzvorrichtung, insbesondere Blendschutzvorrichtung, mit zumindest einem optischen Blendschutzfilter, welcher einen Nasenausschnitt aufweist, mit zumindest einer Sensoreinheit, welche zu einer Erfassung eines Arbeitszustands und einer davon abhängigen Ansteuerung einer Durchlässigkeit, insbesondere einer Lichtdurchlässigkeit, des Blendschutzfilters vorgesehen ist, mit zumindest einer Schildeinheit, in welcher der zumindest eine Blendschutzfilter fest aufgenommen ist, und mit zumindest einer Kopfbefestigungseinheit zu einer Befestigung an einem Kopf eines Benutzers. Vorzugsweise ist der Blendschutzfilter positionsfest in der Schildeinheit aufgenommen.

Die Schutzvorrichtung weist zumindest eine Einstelleinheit auf, durch welche eine Position der zumindest einen Schildeinheit definiert relativ zu der zumindest einen Kopfbefestigungseinheit beweglich ausgeführt ist. Eine Position der zumindest einen Schildeinheit ist durch die Einstelleinheit kontrolliert relativ zu der zumindest einen Kopfbefestigungseinheit beweglich ausgeführt. Unter einer "Schutzvorrichtung" soll in diesem Zusammenhang insbesondere eine Vorrichtung verstanden werden, welche zu einem Schutz eines Benutzers vor zu großer Helligkeit und/oder Funken vorgesehen ist. Vorzugsweise soll darunter insbesondere eine Vorrichtung verstanden werden, welche zu einem Schutz von Augen und/oder einem Gesichtsbereich eines Benutzers während eines Schweiß- und/oder Schleifprozesses dient. Bevorzugt soll darunter insbesondere eine Blendschutzvorrichtung verstanden werden, welche insbesondere zu einem Schutz der Augen eines Benutzers zumindest während eines Schweißprozesses dient. Es sind verschiedene, einem Fachmann als sinnvoll erscheinende Ausgestaltungen einer Schutzvorrichtung denkbar, wie beispielsweise als Schweißhelm, -schirm, -maske und/oder -schild. Ferner soll in diesem Zusammenhang unter einem "optischen Blendschutzfilter" insbesondere ein optischer Filter verstanden werden, welcher insbesondere ein Schutzglas und/oder ein Kunststoffschutzglas bildet. Vorzugsweise soll darunter insbesondere ein optischer Filter verstanden werden, dessen Lichtdurchlässigkeit einstellbar ausgeführt ist. Bevorzugt soll darunter insbesondere ein optischer Schweißschutzfilter mit einer automatischen Verdunkelung verstanden werden. Besonders bevorzugt weist der Blendschutzfilter zumindest eine in der Transmission schaltbare Flüssigkristallebene auf. Es sind verschiedene, einem Fachmann als sinnvoll erscheinende Ausgestaltungen des optischen Blendschutzfilters denkbar, insbesondere soll darunter jedoch ein ADF, auch "automatic darkening filter" oder "automatischer Schweißerschutzfilter" genannt, verstanden werden. Des Weiteren soll unter einem "Nasenausschnitt" in diesem Zusammenhang insbesondere eine immaterielle Aussparung in einem zumindest teilweise transluzenten Teilbereich des Blendschutzfilters verstanden werden, welcher in zumindest einer Betriebsstellung der Schutzvorrichtung zumindest teilweise zu einer zumindest teilweisen Aufnahme einer Nase eines Benutzers vorgesehen ist. Vorzugsweise ist die Aussparung in jedem Punkt in zumindest einer Ebene, insbesondere parallel zu einer Haupterstreckungsebene des Blendschutzfilters, in einem Winkelbereich von zumindest 180° von einem materiellen Teilbereich, insbesondere einem zumindest teilweise transluzenten Teilbereich des Blendschutzfilters umgeben. Vorzugsweise umgreift der Blendschutzfilter in zumindest einer Betriebsstellung der Schutzvorrichtung eine Nase des Benutzers. Besonders bevorzugt beträgt eine vertikale Erstreckung des Nasenausschnitts zumindest 10%, vorzugsweise zumindest 30%, bevorzugt zumindest 50% und besonders bevorzugt zumindest 55% einer vertikalen Erstreckung des Blendschutzfilters. Unter einer "Sensoreinheit" soll in diesem Zusammenhang insbesondere eine Einheit verstanden werden, die dazu vorgesehen ist, zumindest eine Kenngröße und/oder eine physikalische Eigenschaft aufzunehmen, wobei die Aufnahme aktiv, wie insbesondere durch Erzeugen und Aussenden eines elektrischen Messsignals, und/oder passiv, wie insbesondere durch eine Erfassung von Eigenschaftsänderungen eines Sensorbauteils, stattfinden kann. Es sind verschiedene, dem Fachmann als sinnvoll erscheinende Sensoreinheiten denkbar. Vorzugsweise weist die Sensoreinheit insbesondere zumindest eine Fotozelle auf. Bevorzugt ist die Fotozelle insbesondere zumindest zu einer optischen Detektion eines Lichtbogens vorgesehen. Ferner soll in diesem Zusammenhang unter einer "Schildeinheit" insbesondere eine Einheit verstanden werden, welche in einer regulären Betriebsstellung vor einem Gesicht des Benutzers angeordnet ist. Vorzugsweise soll darunter insbesondere eine Einheit verstanden werden, welche in einer Betriebsstellung der Schutzvorrichtung insbesondere zumindest einen wesentlichen Teil eines Gesichts eines Benutzers verdeckt. Vorzugsweise soll darunter insbesondere eine Einheit verstanden werden, welche zu einem Schutz eines Gesichts, wie beispielsweise vor umherfliegenden Funken, vorgesehen ist. Bevorzugt ist die Schildeinheit insbesondere dazu vorgesehen, eine Schutzbarriere zwischen einem Arbeitsbereich und einem Gesicht des Benutzers zu bilden. Des Weiteren soll in diesem Zusammenhang unter einer "Kopfbefestigungseinheit" insbesondere eine Einheit verstanden werden, welche zu einer Befestigung, insbesondere einer Fixierung der Schutzvorrichtung an einem Kopf des Benutzers vorgesehen ist. Vorzugsweise ist die Einheit hierzu dazu vorgesehen, einen Kopf des Benutzers zumindest teilweise zu umgreifen. In diesem Zusammenhang soll unter "zumindest teilweise umgreifen" insbesondere verstanden werden, dass ein Kopf in zumindest einer Ebene in einem Winkelbereich von zumindest 160°, vorzugsweise von zumindest 180°, bevorzugt von zumindest 270° und besonders bevorzugt von zumindest 360° von der Kopfbefestigungseinheit umschlossen wird. Besonders bevorzugt ist die Kopfbefestigungseinheit in einem regulären Betriebszustand dazu vorgesehen zumindest an einer Stirn sowie einem Hinterkopf eines Benutzers anzuliegen. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Ferner soll in diesem Zusammenhang unter einer "Einstelleinheit" insbesondere eine Einheit verstanden werden, mittels welcher insbesondere manuell eine Position der zumindest einen Schildeinheit definiert relativ zu der zumindest einen Kopfbefestigungseinheit einstellbar ausgeführt ist. Vorzugsweise weist die Einstelleinheit zumindest ein Einstellmittel auf, über welches ein Benutzer eine Position und/oder eine Lage der Schildeinheit definiert relativ zu der zumindest einen Kopfbefestigungseinheit verändern kann. Besonders bevorzugt ist die Einstelleinheit insbesondere dazu vorgesehen, eine Bewegung der zumindest einen Schildeinheit definiert relativ zu der zumindest einen Kopfbefestigungseinheit zumindest zu führen. Grundsätzlich ist auch denkbar, dass die Schildeinheit direkt relativ zu der Kopfbefestigungseinheit bewegt wird, wobei eine Bewegung dabei insbesondere durch die Einstelleinheit definiert geführt wird. Dabei soll unter "definiert" insbesondere verstanden werden, dass eine Bewegung der Schildeinheit geführt wird. Vorzugsweise soll darunter zudem verstanden werden, dass eine Grenze einer Bewegung der Schildeinheit relativ zu der zumindest einen Kopfbefestigungseinheit durch die Einstelleinheit vorgegeben ist. Besonders bevorzugt soll darunter insbesondere verstanden werden, dass die Schildeinheit, insbesondere innerhalb der Grenzen der Beweglichkeit, relativ zu der zumindest einen Kopfbefestigungseinheit in mehreren Positionen festlegbar, insbesondere arretierbar ist.

Durch die erfindungsgemäße Schutzvorrichtung kann insbesondere eine vorteilhaft variable Einstellung einer Position der Schildeinheit erreicht werden. Insbesondere kann dadurch erreicht werden, dass die Schildeinheit bedienerspezifisch optimal positioniert werden kann. Hierdurch kann der optische Blendschutzfilter vorzugsweise vorteilhaft klein ausgeführt und/oder ein Sichtfeld vorteilhaft groß ausgeführt werden. Hierdurch kann insbesondere ein vorteilhaft hoher Benutzerkomfort erreicht werden. Ferner kann bei einem großen Sichtfeld insbesondere eine hohe Sicherheit gewährleistet werden. Zudem kann auch durch die optimale Einstellung einer Position der Schildeinheit eine hohe Sicherheit erreicht werden.

Durch die zumindest eine Einstelleinheit ist eine Position der zumindest einen Schildeinheit in zumindest zwei Stellungen relativ zu der zumindest einen Kopfbefestigungseinheit fixierbar ausgeführt. Vorzugsweise ist eine Position der zumindest einen Schildeinheit durch die zumindest eine Einstelleinheit in zumindest zwei differierenden Stellungen relativ zu der zumindest einen Kopfbefestigungseinheit arretierbar. Bevorzugt ist die zumindest eine Schildeinheit insbesondere in einer gewünschten Betriebsstellung fixierbar, insbesondere arretierbar. Besonders bevorzugt ist die zumindest eine Schildeinheit durch die zumindest eine Einstelleinheit, innerhalb der Grenzen der Beweglichkeit, in jeder beliebigen Position und/oder Stellung, insbesondere Betriebsstellung relativ zu der zumindest einen Kopfbefestigungseinheit arretierbar. Unter "fixierbar" soll in diesem Zusammenhang insbesondere verstanden werden, dass eine Relativposition der Schildeinheit, insbesondere manuell, feststellbar bzw. arretierbar ausgebildet ist. Vorzugsweise kann durch eine Fixierung insbesondere ein unbeabsichtigtes Verstellen einer Position und/oder Lage der Schildeinheit, insbesondere allein durch die Gewichtskraft der Schildeinheit selbst, besonders bevorzugt auch bei einer Krafteinwirkung, welche zumindest doppelt so groß wie eine Gewichtskraft der Schildeinheit ist, vermieden werden. Besonders bevorzugt ist eine Ausrichtung und/oder Lage der Schildeinheit fixierbar, wobei eine reine Schwenkbewegung, insbesondere um die Schildeinheit aus einem Bereich vor einem Gesicht des Benutzers zu entfernen, davon insbesondere unabhängig erfolgt. Dadurch kann insbesondere eine vorteilhaft variable Einstellung einer Position der Schildeinheit erreicht werden. Vorzugsweise kann dadurch erreicht werden, dass die Schildeinheit bedienerspezifisch optimal positioniert werden kann. Durch die optimale Einstellung einer Position der Schildeinheit kann insbesondere ein vorteilhaft großes Sichtfeld erreicht werden. Ferner kann hierdurch insbesondere eine Verstellung der Schildeinheit vermieden werden. Des Weiteren ist hierdurch insbesondere eine individuelle Anpassung der Schutzvorrichtung an eine Kopfform des Benutzers möglich.

Durch die zumindest eine Einstelleinheit ist eine Position der zumindest einen Schildeinheit in zumindest zwei Betriebsstellungen relativ zu der zumindest einen Kopfbefestigungseinheit fixierbar ausgeführt. Unter einer "Betriebsstellung" soll in diesem Zusammenhang insbesondere eine Stellung der Schildeinheit verstanden werden, in welcher die Schildeinheit eine vorgesehene Schutzfunktion ausführt und/oder ausführen kann. Vorzugsweise soll darunter insbesondere eine Stellung verstanden werden, in welcher die Schildeinheit vor einem Gesicht eines Benutzers angeordnet ist. Besonders bevorzugt unterscheiden sich die zumindest zwei fixierbaren Betriebsstellungen insbesondere durch eine Entfernung zwischen dem optischen Blendschutzfilter und den Augen des Benutzers und/oder in einem Winkel zwischen der Schildeinheit und der Kopfbefestigungseinheit und/oder in einer Höhe in welcher der optische Blendschutzfilter relativ zu dem Kopf des Benutzers angeordnet ist. Dadurch kann ein Benutzer vorzugsweise zwischen zumindest zwei Betriebsstellungen wählen. Hierdurch kann insbesondere ein vorteilhaft hoher Bedienkomfort erreicht werden. Vorzugsweise kann ein Benutzer bei der Schutzvorrichtung wählen, ob die Schildeinheit auf einer Nase des Benutzers aufliegen soll oder von dieser beabstandet sein soll. Insbesondere kann dadurch zwischen einer Anordnung für Brillenträger und einer Anordnung für Benutzer ohne Brille gewechselt werden. Benutzer ohne Brille können hierdurch ein optimales Sichtfeld erreichen wohingegen Brillenträger einen vorteilhaften Tragekomfort erhalten.

Es wird ferner vorgeschlagen, dass mittels der zumindest einen Einstelleinheit eine direkte Distanz zwischen den Augen des Benutzers und dem optischen Blendschutzfilter einstellbar ausgebildet ist. Vorzugsweise ist mittels der zumindest einen Einstelleinheit eine Distanz zwischen den Augen des Benutzers und dem optischen Blendschutzfilter in einem Betrieb senkrecht zu einer Kopfachse einstellbar. Bevorzugt ist mittels der zumindest einen Einstelleinheit eine Distanz zwischen den Augen des Benutzers und dem optischen Blendschutzfilter in einem Betrieb annähernd senkrecht zu einer Haupterstreckungsebene des Blendschutzfilters einstellbar. Unter einer "direkten Distanz" soll in diesem Zusammenhang insbesondere eine Distanz zwischen den Augen des Benutzers und dem optischen Blendschutzfilter während eines Betriebs, daher bei direktem Blickkontakt, verstanden werden. Dadurch kann insbesondere eine vorteilhaft variable Einstellung einer Position der Schildeinheit erreicht werden. Vorzugsweise kann dadurch erreicht werden, dass die Schildeinheit bedienerspezifisch optimal positioniert werden kann. Je nach Kopfumfang und Wohlbefinden des Schweißer kann dadurch vorteilhaft die Längsposition des optischen Blendschutzfilters eingestellt werden. Ferner kann dadurch eine Längsposition des optischen Blendschutzfilters auch für Brillenträger optimal angepasst werden. Umso näher der optische Blendschutzfilter am Gesicht angeordnet wird, desto größer wird das Sichtfeld für den Benutzer.

Es wird weiter vorgeschlagen, dass mittels der zumindest einen Einstelleinheit in einer Betriebsstellung eine Höhe und/oder ein Winkel der zumindest einen Schildeinheit relativ zu der zumindest einen Kopfbefestigungseinheit einstellbar ausgebildet ist. Vorzugsweise sind/ist eine Höhe und/oder ein Winkel der zumindest einen Schildeinheit insbesondere in einer Ebene parallel zu einer Kopfachse sowie senkrecht zu einer Haupterstreckungsebene des optischen Blendschutzfilters einstellbar ausgebildet. Dadurch kann insbesondere eine vorteilhaft variable Einstellung einer Position und/oder Lage der Schildeinheit erreicht werden. Vorzugsweise kann dadurch erreicht werden, dass die Schildeinheit bedienerspezifisch optimal positioniert werden kann. Durch die optimale Einstellung einer Position der Schildeinheit kann insbesondere ein vorteilhaft großes Sichtfeld erreicht werden.

Ferner wird vorgeschlagen, dass die zumindest eine Schildeinheit zumindest einen Gesichtsanschlag aufweist, welcher in einer Endposition der Schildeinheit dazu vorgesehen ist ein Gesicht des Benutzers zu kontaktieren. Unter einem "Gesichtsanschlag" soll in diesem Zusammenhang insbesondere ein Anschlag verstanden werden, welcher dazu vorgesehen ist, zu einer Definition der Endposition der Schildeinheit relativ zu einem Gesicht des Benutzers, ein Gesicht des Benutzers zu kontaktieren. Vorzugsweise soll darunter insbesondere ein Anschlag verstanden werden, dessen Endposition abhängig von einer Form eines Gesichts des Benutzers ist. Besonders bevorzugt soll darunter insbesondere ein Anschlag verstanden werden, bei welchem eine Anschlagposition durch eine Berührung zwischen Gesicht und Gesichtsanschlag definiert ist. Hierdurch kann zuverlässig eine Endposition der Schildeinheit definiert werden. Ferner kann dadurch zuverlässig die Schildeinheit in eine Betriebsstellung gebracht werden. Insbesondere kann dadurch eine optimale Positionierung der Schildeinheit relativ zu einem Gesicht des Benutzers erreicht werden. Es kann vorzugsweise ein gesichtsspezifischer Anschlag bereitgestellt werden.

Des Weiteren wird vorgeschlagen, dass der zumindest eine Gesichtsanschlag von einer Nasenauflage gebildet ist, welche in zumindest einer Betriebsstellung zu einer Kontaktierung einer Nase eines Benutzers vorgesehen ist. Vorzugsweise ist die Nasenauflage in zumindest einer Betriebsstellung zu einer Kontaktierung eines Nasenrückens eines Benutzers vorgesehen. Bevorzugt ist die Nasenauflage in zumindest einer Betriebsstellung zu einer Kontaktierung einer Nasenpyramide einer Nase eines Benutzers vorgesehen. Besonders bevorzugt ist die Nasenauflage von einer brillenähnlichen Nasenauflage gebildet. Vorzugsweise weist die Nasenauflage zumindest ein Nasenpad auf. Bevorzugt weist die Nasenauflage zumindest zwei Nasenpads auf.

Besonders bevorzugt sind/ist eine Position und/oder eine Lage der Nasenpads einstellbar. Die Nasenauflage ist insbesondere an eine Form einer Nase anpassbar. Hierdurch kann zuverlässig eine Endposition der Schildeinheit definiert werden. Insbesondere kann dadurch eine optimale Positionierung der Schildeinheit relativ zu einem Gesicht des Benutzers erreicht werden. Vorzugsweise kann eine Position der Schildeinheit an einer Gesichtsmitte ausgerichtet werden. Es kann eine zuverlässige Positionierung der Schildeinheit relativ zu den Augen des Benutzers erreicht werden. Zudem kann zumindest ein geringer Teil eines Gewichts der Schutzvorrichtung auf der Nase abgestützt werden. Hierdurch kann insbesondere ein Nach-unten-rutschen der Schutzvorrichtung während eines Betriebs vermieden werden.

Zudem wird vorgeschlagen, dass der zumindest eine Gesichtsanschlag der zumindest einen Schildeinheit zu einer Positionierung der zumindest einen Schildeinheit relativ zu einem Gesicht eines Benutzers vorgesehen ist. Dadurch kann eine zuverlässige Positionierung der Schildeinheit in einer Endposition erreicht werden. Ferner kann dadurch eine optimale Positionierung der Schildeinheit relativ zu einem Gesicht, insbesondere den Augen, des Benutzers erreicht werden. Des Weiteren kann eine Position der Schildeinheit an einer Gesichtsmitte ausgerichtet werden. Insbesondere kann dadurch ein Verrutschen während des Betriebs vermieden werden.

Es wird ferner vorgeschlagen, dass die Kopfbefestigungseinheit in einem regulär getragenen Zustand eine Kopfachse definiert, gegenüber welcher eine Haupterstreckungsebene des optischen Blendschutzfilters in zumindest einer Betriebsstellung wesentlich abgewinkelt ist. Vorzugsweise ist die Haupterstreckungsebene des optischen Blendschutzfilters gegenüber der Kopfachse nach unten abgewinkelt. Unter einer "Kopfachse" soll in diesem Zusammenhang insbesondere eine Achse verstanden werden, welche gegenüber einem Kopf starr ist. Vorzugsweise soll darunter insbesondere eine Achse verstanden werden, welche sich durch einen geometrischen Mittelpunkt des Kopfs des Benutzers erstreckt. Bevorzugt erstreckt sich Kopfachse, bei aufrechter Haltung des Benutzers, parallel zu einer Haupterstreckungsrichtung des Rumpfs des Benutzers. Ferner soll in diesem Zusammenhang unter "wesentlich abgewinkelt" insbesondere verstanden werden, dass ein kleinster Winkel zwischen Haupterstreckungsebene des optischen Blendschutzfilters und der Kopfachse mindestens 5°, vorzugsweise mindestens 10° und besonders bevorzugt mindestens 15° beträgt. Dadurch kann insbesondere ein vorteilhafter, möglichst rechtwinkliger Blickwinkel eines Benutzers auf den optischen Blendschutzfilter erreicht werden. Insbesondere kann durch die Anwinklung des optischen Blendschutzfilters eine senkrechte Sicht des Benutzers durch den optischen Blendschutzfilter ermöglicht werden, was den Einfluss der Blickwinkelabhängigkeit des optischen Blendschutzfilters auf die Sichtqualität reduziert. Insbesondere bei einer natürlichen Arbeitshaltung, bei welcher ein Blick eines Benutzers in der Regel leicht nach unten geneigt ist, kann dadurch der Einfluss der Blickwinkelabhängigkeit des optischen Blendschutzfilters auf die Sichtqualität reduziert werden.

Es wird weiter vorgeschlagen, dass die zumindest eine Kopfbefestigungseinheit von einem Kopfband gebildet ist. Unter einem "Kopfband" soll in diesem Zusammenhang insbesondere eine Kopfbefestigung verstanden werden, welche zumindest ein bandartiges Element umfasst, welches dazu vorgesehen ist, einen Kopf eines Benutzers zumindest teilweise zu umgreifen. Vorzugsweise ist das Kopfband auf eine Größe eines Kopfs eines Benutzers anpassbar. Vorzugsweise kann eine effektive Länge des zumindest einen bandartigen Elements zu einer Anpassung des Kopfbands verändert werden. Hierdurch kann insbesondere eine besonders zuverlässige Kopfbefestigungseinheit bereitgestellt werden. Ferner kann dadurch eine besonders leichte und flexible Kopfbefestigungseinheit bereitgestellt werden.

Ferner wird vorgeschlagen, dass die zumindest eine Schildeinheit aus einem zumindest im Wesentlichen formfesten Material besteht. Vorzugsweise besteht die zumindest eine Schildeinheit aus einem Material mit einem hohen Elastizitätsmodul. Unter einem "hohen Elastizitätsmodul" soll in diesem Zusammenhang insbesondere ein Elastizitätsmodul bei einer Temperatur von 20 °C von zumindest 0,5 kN/mm², vorzugsweise von zumindest 1 kN/mm² und besonders bevorzugt von zumindest 5 kN/mm². Es sind verschiedene, einem Fachmann als sinnvoll erscheinende formfeste Materialien denkbar, wie insbesondere Kunststoff, wie beispielsweise Polycarbonate, Polyamide oder dergleichen, oder Metall. Vorzugsweise soll unter einem formfesten Material insbesondere ein von einem textilen Material differierendes Material verstanden werden. Dadurch kann insbesondere eine vorteilhaft hohe Sicherheit der Schildeinheit erreicht werden. Vorzugsweise kann dadurch ein zuverlässiger Schutz durch die Schildeinheit erreicht werden. Insbesondere kann dadurch eine ungewollte Verformung vermieden werden.

Die erfindungsgemäße Schutzvorrichtung soll hierbei nicht auf die oben beschriebene Anwendung und Ausführungsform beschränkt sein. Insbesondere kann die erfindungsgemäße Schutzvorrichtung zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: die erfindungsgemäße Schutzvorrichtung mit einer Schildeinheit, mit einer Kopfbefestigungseinheit, mit einem optischen Blendschutzfilter und mit einer Vorsatzscheibe auf einem Kopf eines Benutzers in einer schematischen Darstellung,
- Fig. 2: die erfindungsgemäße Schutzvorrichtung mit der Schildeinheit, mit der Kopfbefestigungseinheit und dem optischen Blendschutzfilter in einer schematischen Seitenansicht,
- Fig. 3: die erfindungsgemäße Schutzvorrichtung mit der Schildeinheit, mit der Kopfbefestigungseinheit und dem optischen Blendschutzfilter ohne die Vorsatzscheibe in einer schematischen Frontalansicht,
- Fig. 4: die Kopfbandeinheit der erfindungsgemäßen Schutzvorrichtung in einer schematischen Seitenansicht,
- Fig. 5: die Kopfbandeinheit der erfindungsgemäßen Schutzvorrichtung in einer schematischen Frontalansicht,
- Fig. 6: die Schildeinheit und die Vorsatzscheibe der erfindungsgemäßen Schutzvorrichtung in einer schematischen Seitenansicht, in einer Explosionsdarstellung,
- Fig. 7: der optische Blendschutzfilter der erfindungsgemäßen Schutzvorrichtung in einer schematischen Frontalansicht,
- Fig. 8: die erfindungsgemäße Schutzvorrichtung mit der Schildeinheit, mit der Kopfbefestigungseinheit und dem optischen Blendschutzfilter in einer teilweise transparenten schematischen Seitenansicht während der Einstellung einer Augendistanz,
- Fig. 9: die erfindungsgemäße Schutzvorrichtung mit der Schildeinheit, mit der Kopfbefestigungseinheit und dem optischen Blendschutzfilter in einer teilweise transparenten schematischen Seitenansicht während der Einstellung eines Winkels,
- Fig. 10: die erfindungsgemäße Schutzvorrichtung mit der Schildeinheit, mit der Kopfbefestigungseinheit und dem optischen Blendschutzfilter in einer teilweise transparenten schematischen Seitenansicht während der Einstellung einer Höhe,
- Fig. 11: Sichtfelder der Schutzvorrichtung in zwei verschiedenen Einstellungen im Vergleich zu dem Sichtfeld bereits bekannter Schweißhelme in einer schematischen Darstellung und
- Fig. 12: einen Aufbau einer Messung des Sichtfelds der Schweißhelme in einer schematischen Darstellung.

### Beschreibung des Ausführungsbeispiels

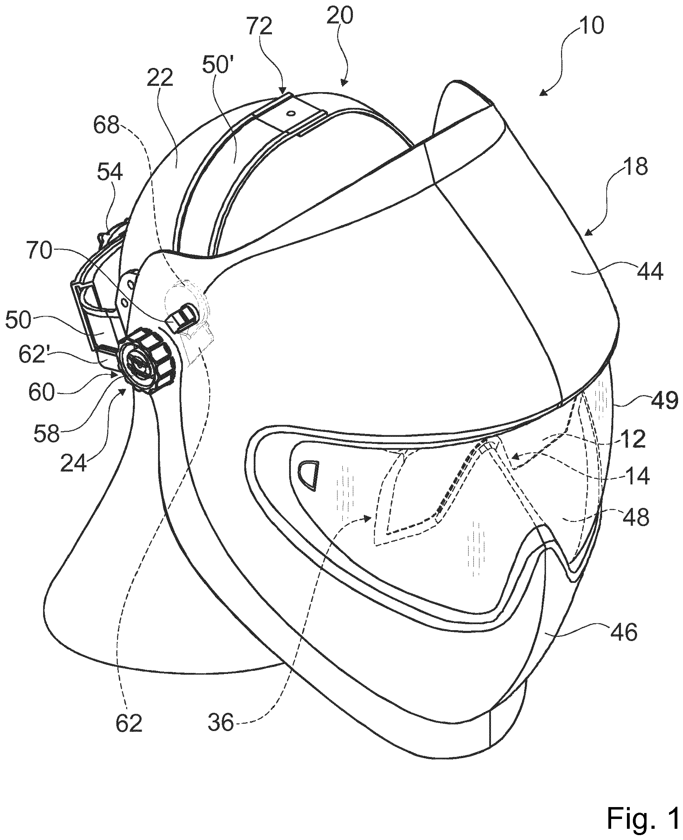

Die Figur 1 zeigt eine Schutzvorrichtung 10 auf einem Kopf 22 eines Benutzers. Die Schutzvorrichtung 10 ist dazu vorgesehen, während eines Betriebs von einem Benutzer auf dem Kopf 22 getragen zu werden. Figur 1 zeigt die Schutzvorrichtung 10 in einer Betriebsstellung. Die Schutzvorrichtung 10 ist von einem Schweißhelm gebildet. Grundsätzlich wäre jedoch auch eine andere, einem Fachmann als sinnvoll erscheinende Ausbildung der Schutzvorrichtung 10 denkbar.

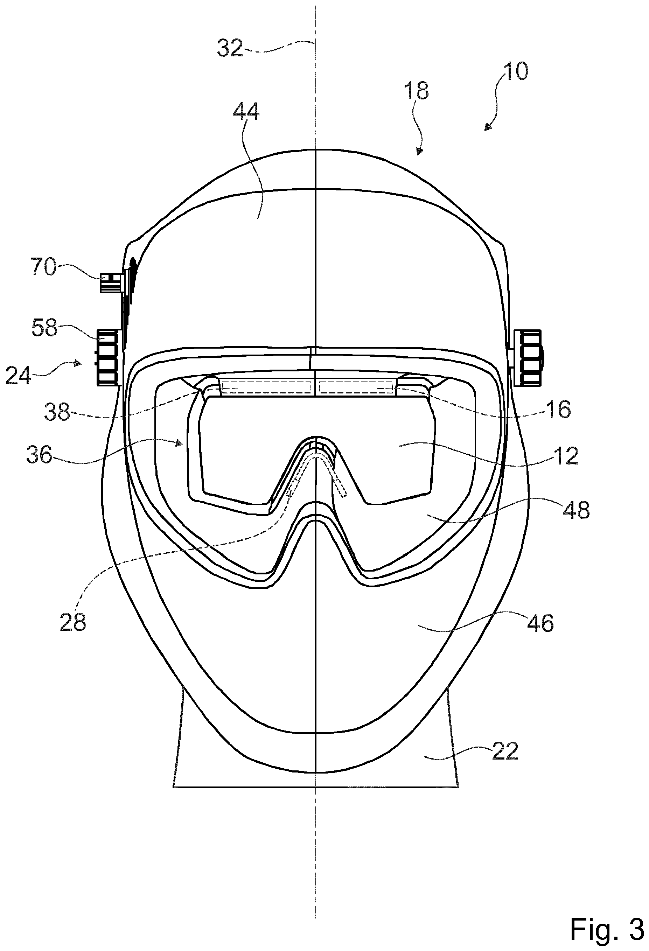

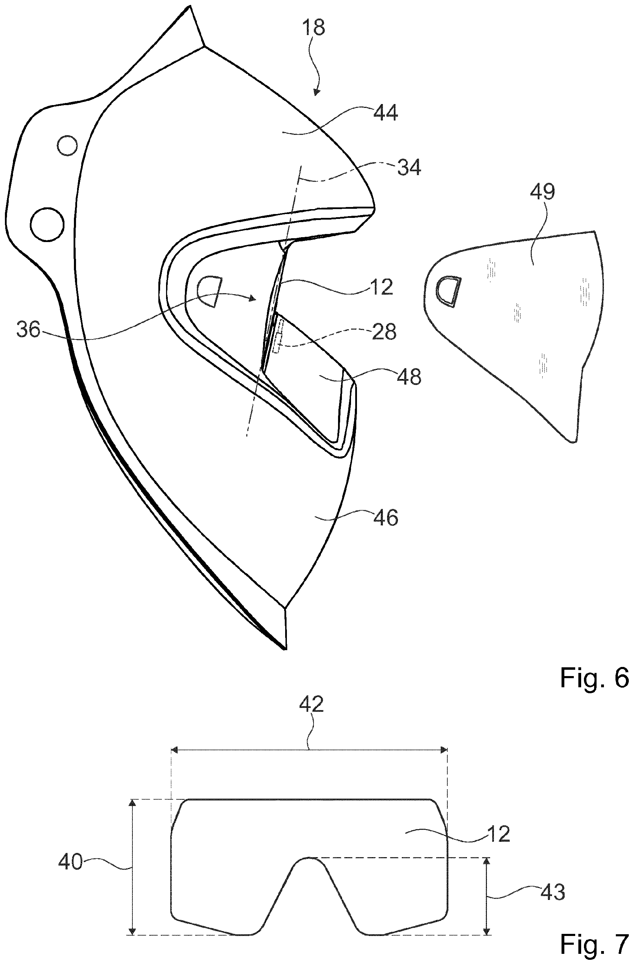

Die Schutzvorrichtung 10 weist einen optischen Blendschutzfilter 12 auf. Ferner weist die Schutzvorrichtung 10 eine Sensoreinheit 16 auf. Die Sensoreinheit 16 ist zu einer Erfassung eines Arbeitszustands und einer davon abhängigen Ansteuerung einer Durchlässigkeit des Blendschutzfilters 12 vorgesehen. Die Sensoreinheit 16 weist zumindest einen Sensor auf, der dazu vorgesehen ist einen Schweißvorgang oder das Auftreten eines hellen Lichts, welches die Augen 26 eines Benutzers schädigen oder auf andere Weise beeinflussen könnte, zu detektieren. Der Sensor der Sensoreinheit 16 ist von einer Fotozelle gebildet. Grundsätzlich wäre jedoch auch einen andere, einem Fachmann als sinnvoll erscheinende Ausbildung des Sensors der Sensoreinheit 16 denkbar. Des Weiteren weist die Schutzvorrichtung 10 eine Schutzkassette 36 auf. Der optische Blendschutzfilter 12 und die Sensoreinheit 16 bilden einen Teil der Schutzkassette 36. Ferner weist die Schutzkassette 36 eine Elektronikeinheit 38 auf. Die Elektronikeinheit 38 ist mit der Sensoreinheit 16 gekoppelt. Die Elektronikeinheit 38 ist dazu vorgesehen, die Daten der Sensoreinheit 16 zu verarbeiten und abhängig davon den Blendschutzfilter 12 anzusteuern. Die Elektronikeinheit 38 umfasst zu einer Energieversorgung eine nicht weiter sichtbare Batterie und/oder Solarzelle auf (Figur 3, 7).

Der optische Blendschutzfilter 12 ist von einem elektrooptischen Filter gebildet. Der optische Blendschutzfilter 12 ist von einem automatic darkening filter, kurz ADF, gebildet. Der optische Blendschutzfilter 12 besteht aus mehreren Schichten. Der optische Blendschutzfilter 12 ist als ein Mehrschichtverbund ausgebildet. Eine Anzahl von Schichten ist hierbei lediglich beispielhaft und kann grundsätzlich variieren. Grundsätzlich wäre auch denkbar, dass der Blendschutzfilter 12 zusätzlich zwischen transparenten Schutzplatten, beispielsweise aus Polycarbonat oder Glas, angeordnet ist, um den Blendschutzfilter 12 zusätzlich zu schützen. Eine Schutzplatte könnte insbesondere dazu genutzt werden den Blendschutzfilter 12 vor Funken und anderen Emissionen des Schweißprozesses zu schützen. Eine entsprechende Schutzplatte ist dabei insbesondere leicht vom Benutzer, insbesondere ohne Verwendung von Werkzeugen, austauschbar. Der optische Blendschutzfilter 12 weist eine Flüssigkristallschicht auf, welche als eine Blende des Blendschutzfilters 12 dient. Die Flüssigkristallschicht ist von einem transluzenten Flüssigkristallbildschirm gebildet. Die Flüssigkristallschicht wird von der Elektronikeinheit 38 angesteuert. Die Flüssigkristallschicht des Blendschutzfilters 12 wird von der Elektronikeinheit 38 aktiviert, wenn über die Sensoreinheit 16 ein Schweißverfahren oder ein Lichtblitz erfasst wird. Die Flüssigkristallschicht des Blendschutzfilters 12 wird von der Elektronikeinheit 38 abgedunkelt, wenn über die Sensoreinheit 16 ein Schweißverfahren oder ein Lichtblitz erfasst wird. Die Flüssigkristallschicht des Blendschutzfilters 12 verringert die Durchlässigkeit sichtbaren Lichts durch den Blendschutzfilter 12. Der optische Blendschutzfilter 12 weist, hinsichtlich einer Lichtdurchlässigkeit, in einem aktiven Zustand beispielhaft eine Schutzstufe von zumindest 5 gemäß DIN auf. Vorzugsweise weist der optische Blendschutzfilter 12 in einem aktiven Zustand beispielhaft einen Schutzstufenbereich zumindest von 4 bis 14 gemäß DIN auf. In einem nicht aktiven Zustand weist der optische Blendschutzfilter 12 hinsichtlich einer Lichtdurchlässigkeit beispielhaft eine Schutzstufe von 4 oder weniger gemäß DIN auf. Die Flüssigkristallschicht ist zwischen zwei Polarisationsschichten des Blendschutzfilters 12 angeordnet. Die Polarisationsschichten sind von Polarisationsfolien gebildet. (Figur 3, 7).

Der Blendschutzfilter 12 weist eine rechteckige Grundform auf. Eine vertikale Erstreckung 40 des Blendschutzfilters 12 ist dabei, in einem regulären Betriebszustand, geringer, als eine horizontale Erstreckung 42 des Blendschutzfilters 12. Die vertikale Erstreckung 40 des Blendschutzfilters 12 beträgt beispielhaft 65 mm. Die horizontale Erstreckung 42 des Blendschutzfilters 12 beträgt 132 mm. Grundsätzlich sind jedoch auch andere einem Fachmann als sinnvoll erscheinende Erstreckungen denkbar. Ferner weist der Blendschutzfilter 12 einen Nasenausschnitt 14 auf. Der Nasenausschnitt 14 ist von einer immateriellen Aussparung in einem materiellen Teil des Blendschutzfilters 12 gebildet. Der Nasenausschnitt 14 ist von einer immateriellen Aussparung in einem materiellen, zumindest teilweise transluzenten Teilbereich des Blendschutzfilters 12 gebildet. Der Nasenausschnitt 14 ist in einer Betriebsstellung der Schutzvorrichtung 10 zu einer teilweisen Aufnahme einer Nase 30 des Benutzers vorgesehen. Der Blendschutzfilter 12 umgreift in einer Betriebsstellung im Bereich des Nasenausschnitts 14 teilweise die Nase 30 des Benutzers. Der Blendschutzfilter 12 weist eine im Wesentlichen rechteckige Grundform auf, wobei der Nasenausschnitt 14 in die rechteckige Grundform ragt. Der Nasenausschnitt 14 weist eine im Wesentlichen dreieckige Form auf. Die beiden durch den materiellen Teil des Blendschutzfilters 12 begrenzten Seiten weisen einen Winkel von annähernd 54° zueinander auf. Die beiden durch den materiellen Teil des Blendschutzfilters 12 begrenzten Seiten laufen an ihren zugewandten Enden über einen Radius zusammen. Die beiden durch den materiellen Teil des Blendschutzfilters 12 begrenzten Seiten laufen an Ihren Enden beispielhaft über einen R9 Radius zusammen. An ihren abgewandten Enden laufen die beiden durch den materiellen Teil des Blendschutzfilters 12 begrenzten Seiten mit einem Radius zu dem materiellen Teil des Blendschutzfilters 12 hin aus. An ihren abgewandten Enden laufen die beiden durch den materiellen Teil des Blendschutzfilters 12 begrenzten Seiten beispielhaft mit einem R7 Radius zu dem materiellen Teil des Blendschutzfilters 12 hin aus. Der Nasenausschnitt 14 erstreckt sich von einer unteren Kante, insbesondere von einer Mitte der unteren Kante des Blendschutzfilters 12 in Richtung eines geometrischen Mittelpunkts des Blendschutzfilters 12. Der Nasenausschnitt 14 ist nach unten hin nicht von dem Blendschutzfilter 12 begrenzt. Eine Form des Nasenausschnitts 14 ist an eine Form einer Nase 30 angepasst. Eine vertikale Erstreckung 43 des Nasenausschnitts 14 beträgt zumindest 45%, vorzugsweise zumindest 50% und besonders bevorzugt zumindest 55% einer vertikalen Erstreckung 40 des Blendschutzfilters 12. Die vertikale Erstreckung 43 des Nasenausschnitts 14 beträgt annähernd 57% einer vertikalen Erstreckung 40 des Blendschutzfilters 12. Die vertikale Erstreckung 43 des Nasenausschnitts 14 beträgt beispielhaft 37 mm. Grundsätzlich ist jedoch auch ein anderer, einem Fachmann als sinnvoll erscheinender Wert der Erstreckung 43 denkbar (Figur 7).

Ferner weist die Schutzvorrichtung 10 eine Schildeinheit 18 auf. Der Blendschutzfilter 12 ist fest in der Schildeinheit 18 aufgenommen. Der Blendschutzfilter 12 ist positionsfest in der Schildeinheit 18 aufgenommen. Der Blendschutzfilter 12 ist in einer Ausnehmung in der Schildeinheit 18 eingepasst. Die gesamte Schutzkassette 36 ist in einer Ausnehmung in der Schildeinheit 18 eingepasst. Die Schildeinheit 18 besteht aus einem im Wesentlichen formfesten Material. Die Schildeinheit 18 besteht aus einem Kunststoff, welcher insbesondere beständig gegenüber Funken und/oder anderen beim Schweißen auftretenden Einflüssen. Die Schildeinheit 18 ist dazu vorgesehen, insbesondere nach den einschlägigen Normen für Schweißmasken, ein Gesicht und/oder Kopf 22 des Benutzers zu bedecken und zu schützen. Die Schildeinheit 18 weist eine teilweise einer Kopfform angepasste Form auf. Die Schildeinheit 18 ist in einem getragenen Zustand der Schutzvorrichtung 10 teilweise um ein Gesicht des Benutzers gebogen. Die Schildeinheit 18 nimmt in einem getragenen Zustand der Schutzvorrichtung 10 teilweise ein Gesicht des Benutzers auf. Die Schildeinheit 18 weist einen Stirnbereich 44, einen Kinnbereich 46 sowie einen zwischen dem Stirnbereich 44 und dem Kinnbereich 46 angeordneten Augenbereich 48 auf. Der Stirnbereich 44, der Kinnbereich 46 und der Augenbereich 48 sind einstückig ausgebildet. Der Stirnbereich 44 und der Kinnbereich 46 sind auf einer dem Gesicht des Benutzers abgewandten Außenseite im Wesentlichen konvex geformt. Der Augenbereich 48 weist eine Aufnahme zur Aufnahme der Schutzkassette 36 auf. Der Augenbereich 48 ist zu der Aufnahme der Schutzkassette 36 hin vertieft. Die Aufnahme für die Schutzkassette 36 ist gegenüber einer Grundform der Schildeinheit 18 nach innen, daher zu einem Gesicht des Benutzers hin, versetzt angeordnet. Die Aufnahme für die Schutzkassette 36 ist gegenüber dem Stirnbereich 44 sowie gegenüber dem Kinnbereich 46 nach innen versetzt angeordnet (Figur 6).

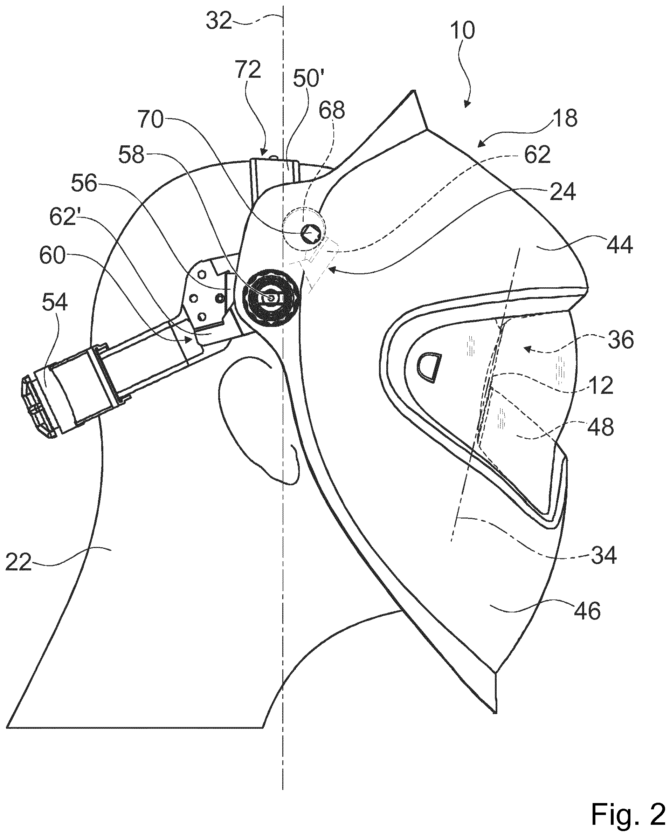

Die Schutzvorrichtung 10 weist ferner eine Vorsatzscheibe 49 auf. Die Vorsatzscheibe 49 ist mit der Schildeinheit 18 über nicht weiter sichtbare Rastelemente verbunden. Vorzugsweise weist die Vorsatzscheibe 49 zwei gegenüberliegende Rastausnehmungen auf, in welche jeweils ein Rastelement der Schildeinheit 18 rastend eingreift. Durch die Verrastung kann die Vorsatzscheibe 49 einfach demontiert werden. Hierdurch sind eine einfache Reinigung und/oder ein einfacher Austausch möglich. Die Vorsatzscheibe 49 ist transparent ausgebildet. Die Vorsatzscheibe 49 ist zu einem Schutz der Schutzkassette 36 vorgesehen. Die Vorsatzscheibe 49 verdeckt die Schutzkassette 36 von außen. Hierdurch kann eine Beschädigung der Schutzkassette 36 vermieden werden. Bei einer Beschädigung der Vorsatzscheibe 49 kann diese, insbesondere im Gegensatz zu der Schutzkassette 36, einfach und kostengünstig ausgetauscht werden. Die Vorsatzscheibe 49 deckt einen wesentlichen Teil des Augenbereichs 48 der Schildeinheit 18 ab. Die Vorsatzscheibe 49 liegt an einer umlaufenden Kante vollständig an der Schildeinheit 18 an. Grundsätzlich ist denkbar, dass an der Schildeinheit 18 und/oder der Vorsatzscheibe 49 eine Dichtung vorgesehen ist, welche beispielsweise das Eindringen von Staub und/oder Funken in einen Zwischenraum zwischen der Schutzkassette 36 und der Vorsatzscheibe 49 verhindert. Die Vorsatzscheibe 49 deckt im Wesentlichen die Vertiefung des Augenbereichs 48 ab. Die Vorsatzscheibe 49 ist entsprechend einer Grundform der Schildeinheit 18 gebogen (Figur 2, 6).

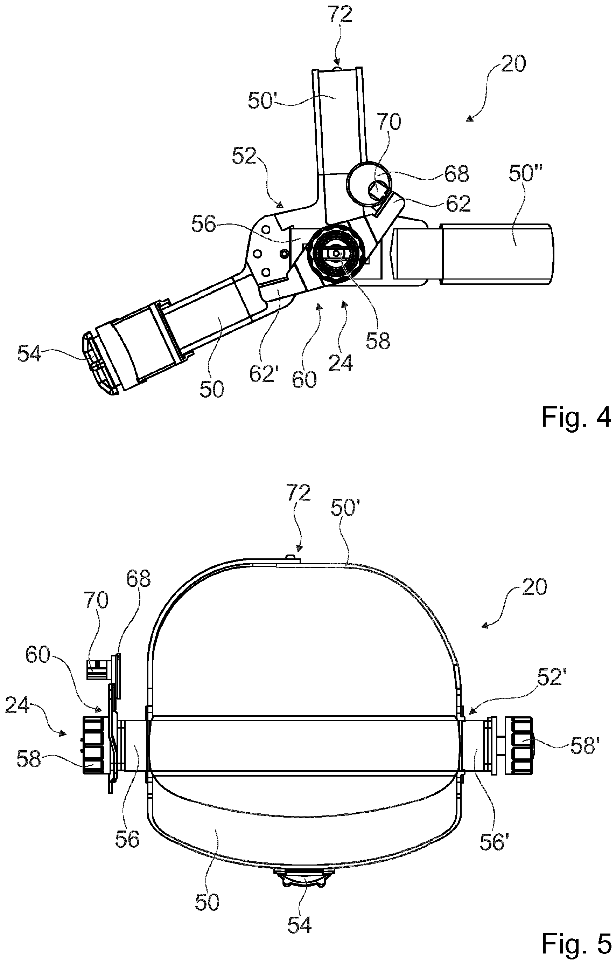

Des Weiteren weist die Schutzvorrichtung 10 eine Kopfbefestigungseinheit 20 auf. Die Kopfbefestigungseinheit 20 ist zu einer Befestigung an dem Kopf 22 des Benutzers vorgesehen. Die Kopfbefestigungseinheit 20 ist von einem Kopfband gebildet. Die Kopfbefestigungseinheit 20 weist mehrere Kopfbandelemente 50, 50', 50" auf, die jeweils dazu vorgesehen sind, sich zumindest teilweise um einen Kopf 22 des Benutzers zu erstrecken. Die Kopfbefestigungseinheit 20 weist drei Kopfbandelemente 50, 50', 50" auf, die jeweils dazu vorgesehen sind, sich in verschiedenen Ebenen um den Kopf 22 des Benutzers zu erstrecken. Die drei Kopfbandelemente 50, 50', 50" sind bandförmig ausgebildet. Ein erstes Kopfbandelement 50 erstreckt sich von einem ersten gemeinsamen Verbindungspunkt 52 oberhalb eines Ohrs des Benutzers um einen Hinterkopf des Kopfes 22 des Benutzers zu einem zweiten gemeinsamen Verbindungspunkt 52' oberhalb des anderen Ohrs des Benutzers. Eine effektive Länge des ersten Kopfbandelements 50 ist einstellbar. Die Kopfbefestigungseinheit 20 weist an dem Kopfbandelement 50 ein Einstellelement 54 auf. Das Einstellelement 54 ist von einem Einstellrad gebildet, welches beispielsweise von Helmen bekannt ist, mittels welchem durch Drehen eine effektive Länge des ersten Kopfbandelements 50 eingestellt werden kann. Ein zweites Kopfbandelement 50' erstreckt sich von dem ersten gemeinsamen Verbindungspunkt 52 über einen Oberkopf des Kopfes 22 des Benutzers zu dem zweiten gemeinsamen Verbindungspunkt 52' oberhalb des anderen Ohrs des Benutzers. Das zweite Kopfbandelement 50' erstreckt sich von dem ersten gemeinsamen Verbindungspunkt 52 annähernd in einem Winkel von 120° gegenüber dem ersten Kopfbandelement 50. Eine effektive Länge des zweiten Kopfbandelements 50' ist einstellbar. Das zweite Kopfbandelement 50' ist dazu zweiteilig ausgebildet. Die zwei Teile des zweiten Kopfbandelements 50' sind über eine verstellbare Verschlusseinheit 72 verbunden. Die verstellbare Verschlusseinheit 72 ist von einem Snapback-Verschluss gebildet. Ein drittes Kopfbandelement 50" erstreckt sich von dem ersten gemeinsamen Verbindungspunkt 52 um eine Stirn des Kopfes 22 des Benutzers zu dem zweiten gemeinsamen Verbindungspunkt 52' oberhalb des anderen Ohrs des Benutzers. Das dritte Kopfbandelement 50" erstreckt sich von dem ersten gemeinsamen Verbindungspunkt 52 annähernd in einem Winkel von 90° gegenüber dem zweiten Kopfbandelement 50'. Die drei Kopfbandelemente 50, 50', 50" der Kopfbefestigungseinheit 20 sind über die Verbindungspunkte 52, 52' fest miteinander verbunden. Die Kopfbefestigungseinheit 20 besteht aus einem teilweise elastischen Kunststoff. Grundsätzlich wäre jedoch auch ein anderes, einem Fachmann als sinnvoll erscheinendes Material denkbar (Figur 2, 4, 5).

Die Kopfbefestigungseinheit 20 definiert in einem regulär getragenen Zustand eine Kopfachse 32. Die Kopfachse 32 ist gegenüber dem Kopf 22 des Benutzers starr. Die Kopfachse 32 beschreibt eine Lage des Kopfes 22. Die Kopfachse 32 erstreckt sich durch einen geometrischen Mittelpunkt des Kopfs 22 des Benutzers. Ferner erstreckt sich die Kopfachse 32, bei aufrechter Haltung des Benutzers, parallel zu einer Haupterstreckungsrichtung des Rumpfs des Benutzers. Des Weiteren erstreckt sich die Kopfachse 32 zumindest annähernd parallel zu einer Erstreckungsebene des zweiten Kopfbandelements 50' (Figur 2, 3).

Ferner weist die Schutzvorrichtung 10 eine Einstelleinheit 24 auf. Mittels der Einstelleinheit 24 ist eine Position der Schildeinheit 18 definiert relativ zu der Kopfbefestigungseinheit 20 beweglich ausgeführt. Mittels der Einstelleinheit 24 kann eine Position der Schildeinheit 18 definiert relativ zu der Kopfbefestigungseinheit 20 verstellt werden. Durch die Einstelleinheit 24 ist eine Position der Schildeinheit 18 in zumindest zwei Stellungen relativ zu der zumindest einen Kopfbefestigungseinheit 20 fixierbar ausgeführt. Durch die Einstelleinheit 24 ist eine Position der Schildeinheit 18 in zumindest zwei Betriebsstellungen relativ zu der zumindest einen Kopfbefestigungseinheit 20 fixierbar ausgeführt. Mittels der Einstelleinheit 24 ist eine Position der Schildeinheit 18 in einem begrenzten Bereich in einer beliebigen Betriebsstellung relativ zu der zumindest einen Kopfbefestigungseinheit 20 fixierbar ausgeführt. Mittels der Einstelleinheit 24 kann eine Lage der Schildeinheit 18 in einer Betriebsstellung frei gewählt werden, wobei die Lage der Schildeinheit 18 fixiert werden kann. Die Schildeinheit 18 ist über die Einstelleinheit 24 mit der Kopfbefestigungseinheit 20 verbunden. Die Einstelleinheit 24 ist an den zwei Verbindungspunkten 52, 52' mit der Kopfbefestigungseinheit 20 verbunden. Ferner ist die Einstelleinheit 24 in einem Randbereich des Stirnbereichs 44 der Schildeinheit 18 mit der Schildeinheit 18 verbunden. Die Einstelleinheit 24 umfasst ein Grundelement 56. Das Grundelement 56 ist an dem ersten Verbindungspunkt 52 der Kopfbefestigungseinheit 20 fest mit der Kopfbefestigungseinheit 20 verbunden. Ferner umfasst die Einstelleinheit 24 ein zu dem Grundelement 56 spiegelsymmetrisches zweites Grundelement 56'. Das zweite Grundelement 56' ist an dem zweiten Verbindungspunkt 52' der Kopfbefestigungseinheit 20 fest mit der Kopfbefestigungseinheit 20 verbunden (Figur 2).

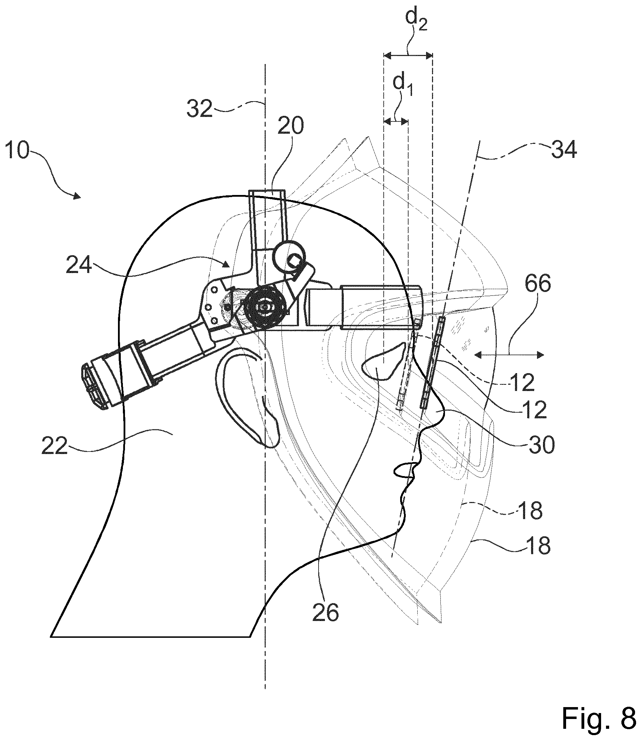

Ferner weist die Einstelleinheit 24 ein Drehrad 58 auf, welches drehbar an der Schildeinheit 18 gelagert ist. Ferner ist das Drehrad 58 in einer Führungsschiene des ersten Grundelements 56 translatorisch geführt. Das Drehrad 58 ist in der Führungsschiene des ersten Grundelements 56 senkrecht zu der Kopfachse 32 geführt. In der Führungsschiene des ersten Grundelements 56 ist ein nicht weiter sichtbares Führungselement geführt, an welchem das Drehrad 58 angeordnet ist. Das Führungselement ist von einer Vierkantmutter gebildet, in welcher das Drehrad 58 eingeschraubt ist. Eine Position des Drehrads 58 relativ zu der Führungsschiene des Grundelements 56 kann von einem Benutzer durch Verdrehen fixiert oder gelöst werden. In einem gelösten Zustand kann das Drehrad 58 in der Führungsschiene verschoben werden. Auf einer dem Drehrad 58 gegenüberliegenden Seite der Kopfbefestigungseinheit 20 ist ein identisches zweites Drehrad 58' angeordnet, welches entsprechend in einer Führungsschiene des zweiten Grundelements 56' translatorisch geführt ist. Mittels der Einstelleinheit 24 ist eine direkte Distanz d₁, d₂ zwischen den Augen 26 des Benutzers und dem optischen Blendschutzfilter 12 einstellbar ausgebildet. Mittels der Einstelleinheit 24 kann eine direkte Distanz d₁, d₂ zwischen den Augen 26 des Benutzers und dem optischen Blendschutzfilter 12 in horizontaler Richtung bzw. senkrecht zu der Kopfachse 32 eingestellt werden. Die direkte Distanz d₁, d₂ zwischen den Augen 26 des Benutzers und dem optischen Blendschutzfilter 12 kann mittels den Drehrädern 58, 58' der Einstelleinheit 24 verändert werden. Mittels der Drehräder 58, 58' kann eine Position der Schildeinheit 18 in einer Längsrichtung 66 relativ zu dem Gesicht des Benutzers verändert werden. Ist eine gewünschte Längsposition gefunden kann die Position durch Verdrehen der Drehräder 58, 58' fixiert werden. Je nach Kopfumfang und Wohlbefinden des Schweißer kann dadurch vorteilhaft die Längsposition der Schutzkassette 36 eingestellt werden. Insbesondere kann dadurch eine Längsposition der Schutzkassette 36 auch für Brillenträger optimal angepasst werden. Umso näher die Schutzkassette 36 am Gesicht angeordnet wird, desto größer wird das Sichtfeld 74 für den Benutzer. Ferner bilden die Drehrädern 58, 58' ein Drehgelenk der Schildeinheit 18. Die Schildeinheit 18 ist an den Drehrädern 58, 58' drehbar gelagert. Über die Drehräder 58, 58' kann die Schildeinheit 18 nach oben geschwenkt werden. Hierdurch kann die Schildeinheit 18 aus einem Bereich des Gesichts des Kopfs 22 des Benutzers entfernt werden, ohne die Schutzvorrichtung 10 auszuziehen. In der Figur 8 sind beispielhaft zwei verschiedene Distanzen d₁, d₂ zwischen den Augen 26 des Benutzers und dem optischen Blendschutzfilter 12 dargestellt, welche mittels der Einstelleinheit 24 eingestellt werden können. Eine Einstellung der Distanz d₁, d₂ zwischen den Augen 26 des Benutzers und dem optischen Blendschutzfilter 12 ist stufenlos möglich (Figur 2, 4, 8).

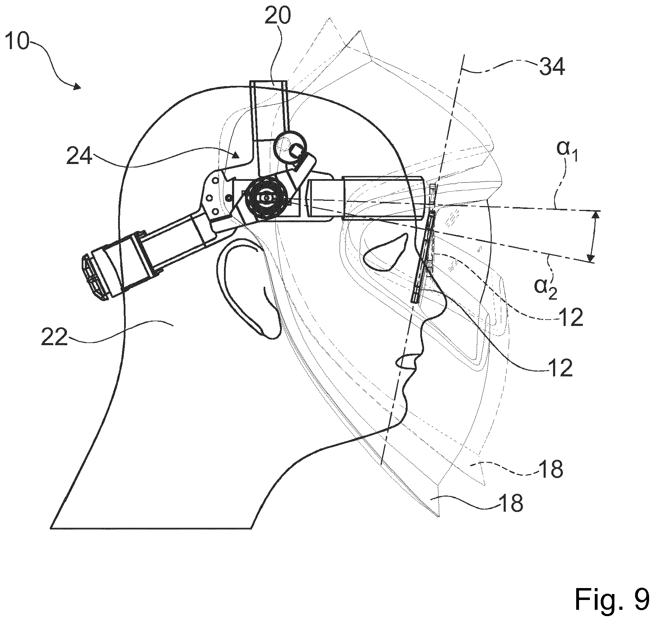

Ferner ist mittels der Einstelleinheit 24 in einer Betriebsstellung ein Winkel α₁, α₂ der Schildeinheit 18 relativ zu der Kopfbefestigungseinheit 20 einstellbar ausgebildet. Die Einstelleinheit 24 weist dazu einen Hebel 60 auf. Der Hebel 60 ist fest mit dem in der Führungsschiene des ersten Grundelements 56 geführten Führungselement verbunden. Der Hebel 60 ist fest mit der Vierkantmutter verbunden, in welche das Drehrad 58 eingeschraubt ist. Der Hebel 60 weist daher gegenüber der Kopfbefestigungseinheit 20 eine feste Winkellage auf. Der Hebel 60 weist zwei Hebelarme 62, 62' auf, die auf gegenüberliegenden Seiten einer Drehachse des Drehrads 50 angeordnet sind. Die Hebelarme 62, 62' bilden jeweils einen Anschlag. Die Hebelarme 62, 62' erstrecken sich jeweils in einer Ebene parallel zu der Kopfachse 32 und senkrecht zu einer Haupterstreckungsebene 34 des optischen Blendschutzfilters 12. Ein erster Hebelarm 62 erstreckt sich von der Schwenkachse aus in eine dem optischen Blendschutzfilter 12 zugewandten Richtung. Der erste Hebelarm 62 weist einen korrespondierenden Anschlag auf. Der korrespondierende Anschlag ist von einer exzentrisch gelagerten Scheibe 68 gebildet. Die exzentrisch gelagerte Scheibe 68 ist kreisrund ausgebildet. Die exzentrisch gelagerte Scheibe 68 ist über ein Verstellrad 70 drehbar an der Schildeinheit 18 gelagert. Das Verstellrad 70 ist oberhalb des Drehrads 58 an der Schildeinheit 18 angeordnet. An der Schildeinheit 18 ist auf einer Innenseite um das Verstellrad 70 ein Zahnkranz angeordnet, mit welchem das Verstellrad 70 in den einzelnen Verstellpositionen verrastet. Dadurch kann ein ungewolltes Verstellen des Verstellrads 70 vermieden werden. Zudem kann dadurch bei einer Verstellung ein akustische Rückmeldung gegeben. Über das Verstellrad 70 kann in einer Betriebsstellung ein Winkel α₁, α₂ der Schildeinheit 18 relativ zu der Kopfbefestigungseinheit 20 eingestellt werden. Über das Verstellrad 70 kann ein Winkel des optischen Blendschutzfilters 12 relativ zu der Kopfachse 32 eingestellt werden. Der erste Hebelarm 62 liegt abhängig von einer Drehstellung des Verstellrads 70 an unterschiedlichen Punkten eines Umfangs der Scheibe 68 an. Abhängig von einem Punkt des Umfangs der Scheibe 68, an welchem der erste Hebelarm 62 anliegt, verändert sich aufgrund der Exzentrizität der Scheibe 68 eine Entfernung zwischen dem Hebelarm 62' und einer Drehachse der Scheibe 68. Hierdurch wird ein Winkel zwischen der Schildeinheit 18 und dem Hebel 60 verändert. Der Hebel 60 weist gegenüber der Kopfbefestigungseinheit 20 eine feste Winkellage auf. Hieraus ergibt sich eine Veränderung des Winkels α₁, α₂ der Schildeinheit 18 relativ zu der Kopfbefestigungseinheit 20. Die Scheibe 68 wird durch ein Gewicht der Schildeinheit 18 gegen den ersten Hebelarm 62 des Hebels 60 gedrückt. Dadurch kann Winkel des optischen Blendschutzfilters 12 an die jeweilige Gesichtsform des Benutzers angepasst werden. In einer entsprechenden Anschlagsposition befindet sich die Schildeinheit 18 in einer Betriebsposition. Ein zweiter Hebelarm 62' erstreckt sich von der Schwenkachse aus in eine von dem optischen Blendschutzfilter 12 abweisende Richtung. Der zweite Hebelarm 62' weist einen korrespondierenden Anschlag auf. Der korrespondierende Anschlag ist ebenfalls von der exzentrisch gelagerten Scheibe 68 gebildet. Der zweite Hebelarm 62' bildet einen Anschlag der Schildeinheit 18 in einem nach oben geschwenkten Zustand, in welchem die Schildeinheit 18 aus einem Bereich des Gesichts des Kopfs 22 des Benutzers entfernt angeordnet ist. Die Scheibe 68 wird in einem nach oben geschwenkten Zustand der Schildeinheit 18 ebenfalls durch ein Gewicht der Schildeinheit 18 gegen den zweiten Hebelarm 62' des Hebels 60 gedrückt. In einer entsprechenden Anschlagsposition befindet sich die Schildeinheit 18 in einer Ruheposition. In der Figur 9 sind beispielhaft zwei verschiedene Winkel α₁, α₂ der Schildeinheit 18 dargestellt, welche mittels der Einstelleinheit 24 eingestellt werden können. Eine Einstellung des Winkels α_{1,} α₂ der Schildeinheit 18 ist stufenlos möglich. In der Figur 9 sind beispielhaft zwei verschiedene Winkel α₁, α₂ der Schildeinheit 18 dargestellt, welche mittels der Einstelleinheit 24 in einer Betriebsposition eingestellt werden können (Figur 2, 4, 9).

Die Haupterstreckungsebene 34 des optischen Blendschutzfilters 12 ist in zumindest einer Betriebsstellung gegenüber der Kopfachse 32 wesentlich abgewinkelt. Bei einer maximalen Winkelstellung der Schildeinheit 18 relativ zu der Kopfbefestigungseinheit 20 mit dem Winkel α₁ ist die Haupterstreckungsebene 34 des optischen Blendschutzfilters 12 gegenüber der Kopfachse 32 wesentlich abgewinkelt. Ein kleinster Winkel zwischen der Haupterstreckungsebene 34 des optischen Blendschutzfilter 12 und der Kopfachse 32 beträgt in der maximalen Winkelstellung der Schildeinheit 18 mindestens 5°, vorzugsweise mindestens 10°. Der kleinste Winkel beträgt annähernd 15°. Die Haupterstreckungsebene 34 des optischen Blendschutzfilters 12 ist dabei derart gegenüber der Kopfachse 32 abgewinkelt, dass eine untere Kante des optischen Blendschutzfilters 12, in welcher der Nasenausschnitt 14 eingebracht ist, näher an der Kopfachse 32 befindet, als eine obere Kante des optischen Blendschutzfilters 12. Durch die Anwinklung des optischen Blendschutzfilters 12 kann eine senkrechte Sicht des Benutzers durch den optischen Blendschutzfilter 12 ermöglicht werden, was den Einfluss der Blickwinkelabhängigkeit des optischen Blendschutzfilters 12 auf die Sichtqualität reduziert (Figur 9).

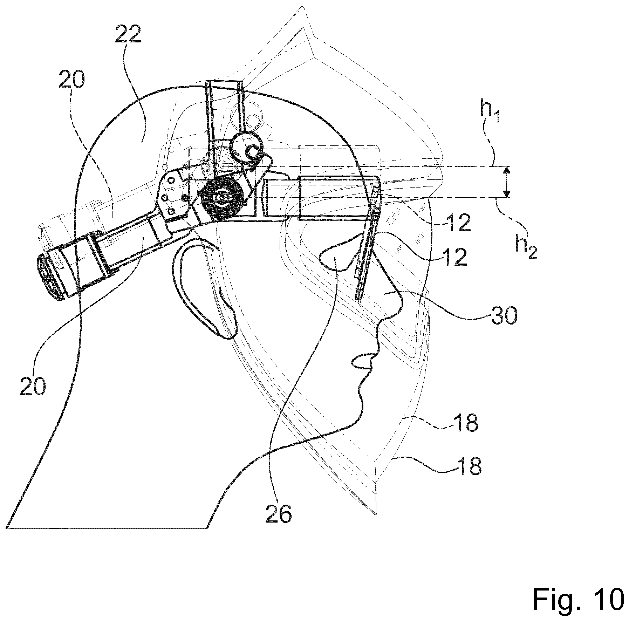

Des Weiteren ist mittels der Einstelleinheit 24 in einer Betriebsstellung eine Höhe h₁, h₂ der Schildeinheit 18 relativ zu der Kopfbefestigungseinheit 20 einstellbar ausgebildet. Mittels der Einstelleinheit 24 ist in einer Betriebsstellung eine Höhe h₁, h₂ der Schildeinheit 18 parallel zu der Kopfachse 32 relativ zu dem Kopf 22 des Benutzers einstellbar ausgebildet. Die Einstelleinheit 24 weist dazu die verstellbare Verschlusseinheit 72 auf. Die verstellbare Verschlusseinheit 72 ist dazu vorgesehen die zwei Teile des zweiten Kopfbandelements 50' zu verbinden. Die verstellbare Verschlusseinheit 72 ist von einem Snapback-Verschluss gebildet. Die verstellbare Verschlusseinheit 72 ist einstückig mit dem zweiten Kopfbandelement 50' ausgebildet. Mittels der verstellbaren Verschlusseinheit 72 kann eine effektive Länge des zweiten Kopfbandelements 50' verändert werden. Mittels der verstellbaren Verschlusseinheit 72 kann durch den Benutzer manuell eine effektive Länge des zweiten Kopfbandelements 50' verändert werden. Durch die Veränderung der effektiven Länge des zweiten Kopfbandelements 50' kann eine Höhe der Verbindungspunkte 52, 52' parallel zu der Kopfachse 32 relativ zu dem Kopf 22 des Benutzers eingestellt werden. Hierdurch kann wiederum die Höhe h₁, h₂ der Schildeinheit 18 eingestellt werden. In der Figur 10 sind beispielhaft zwei verschiedene Höhe h₁, h₂ der Schildeinheit 18 dargestellt, welche mittels der Einstelleinheit 24 eingestellt werden können. Eine Anzahl einstellbarer Höhen h₁, h₂ hängt dabei von einer Ausgestaltung der verstellbaren Verschlusseinheit 72 ab. Grundsätzlich wäre jedoch auch eine stufenlose Einstellbarkeit der Verschlusseinheit 72 und damit auch der Höhe h₁, h₂ denkbar (Figur 2, 4, 10).

Die Schildeinheit 18 weist ferner einen Gesichtsanschlag 28 auf. Der Gesichtsanschlag 28 ist in einer Endposition der Schildeinheit 18 dazu vorgesehen, ein Gesicht des Benutzers zu kontaktieren. Eine Anschlagposition ist dabei durch eine Berührung zwischen Gesicht und Gesichtsanschlag 28 definiert. Eine Anschlagsposition definiert dabei eine Betriebsstellung. Der Gesichtsanschlag 28 der Schildeinheit 18 ist zu einer Positionierung der Schildeinheit 18 relativ zu einem Gesicht eines Benutzers vorgesehen. Der Gesichtsanschlag 28 ist von einer Nasenauflage gebildet. Der Gesichtsanschlag 28 ist von einer brillenähnlichen Nasenauflage gebildet. Der Gesichtsanschlag 28 ist in einer Betriebsstellung zu einer Kontaktierung einer Nase 30 des Benutzers vorgesehen. Der Gesichtsanschlag 28 ist in einer Betriebsstellung zu einer Kontaktierung eines Nasenrückens der Nase 30 des Benutzers vorgesehen. Der Gesichtsanschlag 28 weist zwei Nasenpads auf, welche in einer Betriebsstellung dazu vorgesehen sind jeweils eine Seite der Nase 30 zu kontaktieren. Eine Position und Lage der Nasenpads des Gesichtsanschlags 28 ist einstellbar. Die Nasenpads des Gesichtsanschlags 28 sind dazu über verformbare Verbindungsstege mit einem Grundkörper der Schildeinheit 18 verbunden. Der Gesichtsanschlags 28 ist dadurch an eine Form einer Nase 30 anpassbar. Hierdurch kann zuverlässig eine Endposition der Schildeinheit 18 definiert werden. Insbesondere kann dadurch eine zuverlässige Positionierung der Schildeinheit 18 relativ zu den Augen 26 des Benutzers erreicht werden. Der Gesichtsanschlag 28 ist in einem Bereich des Nasenausschnitts 14 des optischen Blendschutzfilters 12 angeordnet. Grundsätzlich wäre jedoch auch eine andere, einem Fachmann als sinnvoll erscheinende Ausgestaltung und/oder Anordnung des Gesichtsanschlags 28 denkbar (Figur 3).

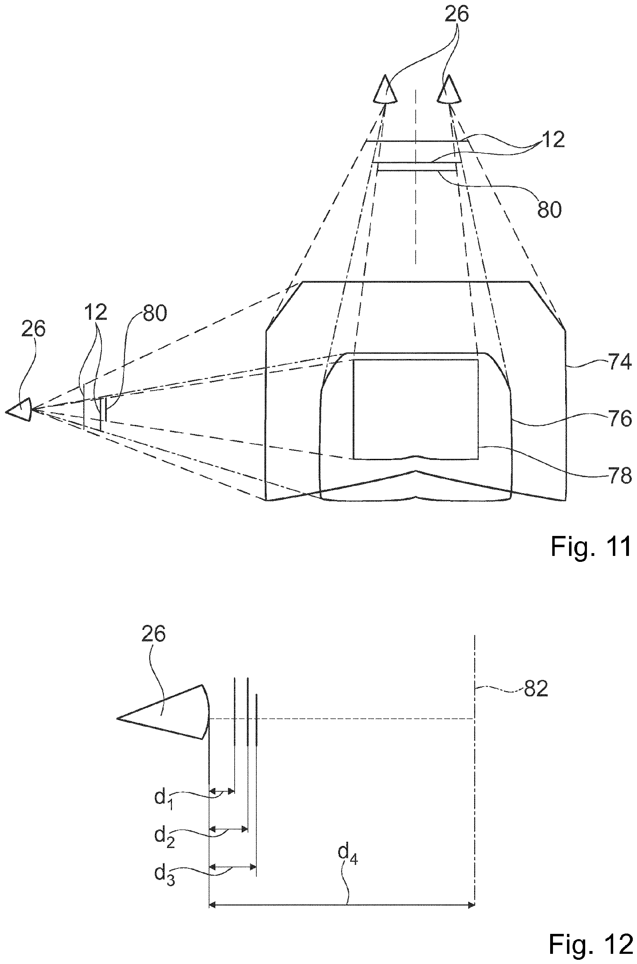

Figur 11 zeigt zwei mögliche Sichtfelder 74, 76 der Schutzvorrichtung 10 in zwei verschiedenen Einstellungen im Vergleich zu dem Sichtfeld 78 bereits bekannter Schweißhelme, wie hier beispielhaft das Sichtfeld 78 eines Schweißhelms der Optrel AG mit der Bezeichnung "e684". Ein optischer Blendschutzfilter 80 des Schweißhelms mit der Bezeichnung "e684" weist eine rechteckige Standardgröße von 50 mm x 100 mm auf. Der optische Blendschutzfilter 80 des Schweißhelms mit der Bezeichnung "e684" weist daher eine Fläche von 50 cm². Der optische Blendschutzfilter 12 der Schutzvorrichtung 10 weist eine Fläche von circa 71 cm² auf. Die Sichtfelder 74, 76, 78 werden dabei in einer Ebene 82 parallel zu den Blendschutzfiltern 12, 80 und mit einer Entfernung d₄ von 600 mm zu einem Augapfel eines Benutzers der Schutzvorrichtung 10 bzw. des Schweißhelms mit der Bezeichnung "e684" gemessen (Figur 12). Es wird das Stereosehen gemessen. Das erste Sichtfeld 74 der Schutzvorrichtung 10 in einer ersten Einstellung weist in der Ebene 82 eine Fläche von 6946 cm² auf. In der ersten Einstellung der Schutzvorrichtung 10 ist der optische Blendschutzfilter 12 mittels der Einstelleinheit 24 auf eine minimale Distanz d₁ zu den Augen des Benutzers eingestellt. Die Distanz d₁ beträgt circa 20 mm. Das zweite Sichtfeld 76 der Schutzvorrichtung 10 in einer zweiten Einstellung weist in der Ebene 82 eine Fläche von 2525 cm² auf. In der zweiten Einstellung der Schutzvorrichtung 10 ist der optische Blendschutzfilter 12 mittels der Einstelleinheit 24 auf eine maximale Distanz d₂ zu den Augen des Benutzers eingestellt. Die Distanz d₂ beträgt circa 40 mm. Das Sichtfeld 78 des Schweißhelms mit der Bezeichnung "e684" weist in einem regulär getragenen Zustand in der Ebene 82 eine Fläche von 1090 cm² auf. Das Sichtfeld 78 des Schweißhelms mit der Bezeichnung "e684" weist in einem regulär getragenen Zustand eine Distanz d₃ von circa 50 mm zu den Augen des Benutzers auf. Das Sichtfeld 74, 76 der Schutzvorrichtung 10 in der Ebene 82 ist daher zumindest 200%, vorzugsweise in zumindest einer Einstellung zumindest 400% und besonders bevorzugt in zumindest einer Einstellung, insbesondere einer optimalen Einstellung, zumindest 600% größer als das Sichtfeld 78 des bereits bekannten Schweißhelms. Ferner ist das Sichtfeld 74, 76 der Schutzvorrichtung 10 in der Ebene 82 relativ zu einer Größe der optischen Blendschutzfilter 12, 80 zumindest 150%, vorzugsweise in zumindest einer Einstellung zumindest 250% und besonders bevorzugt in zumindest einer Einstellung zumindest 500% größer als das Sichtfeld 78 des bereits bekannten Schweißhelms. Die Fläche des Sichtfelds 74, 76 der Schutzvorrichtung 10 ist in der Ebene 82 zumindest 2-mal, vorzugsweise in zumindest einer Einstellung zumindest 3-mal und besonders bevorzugt in zumindest einer Einstellung, insbesondere einer optimalen Einstellung, zumindest 5-mal größer als eine Fläche des optischen Blendschutzfilters 12 der Schutzvorrichtung 10.

## Patentansprüche

1. Schutzvorrichtung, insbesondere Blendschutzvorrichtung, mit zumindest einem optischen Blendschutzfilter (12), welcher einen Nasenausschnitt (14) aufweist, mit zumindest einer Sensoreinheit (16), welche zu einer Erfassung eines Arbeitszustands und einer davon abhängigen Ansteuerung einer Durchlässigkeit des Blendschutzfilters (12) vorgesehen ist, mit zumindest einer Schildeinheit (18), in welcher der zumindest eine Blendschutzfilter (12) fest aufgenommen ist, und mit zumindest einer Kopfbefestigungseinheit (20) zu einer Befestigung an einem Kopf (22) eines Benutzers, **gekennzeichnet durch** zumindest eine Einstelleinheit (24), durch welche eine Position der zumindest einen Schildeinheit (18) definiert relativ zu der zumindest einen Kopfbefestigungseinheit (20) beweglich ausgeführt ist, wobei durch die zumindest eine Einstelleinheit (24) eine Position der zumindest einen Schildeinheit (18) in zumindest zwei Betriebsstellungen relativ zu der zumindest einen Kopfbefestigungseinheit (20) fixierbar ausgeführt ist.

2. Schutzvorrichtung nach Anspruch 1,
**dadurch gekennzeichnet, dass**
durch die zumindest eine Einstelleinheit (24) eine Position der zumindest einen Schildeinheit (18) in zumindest zwei Stellungen relativ zu der zumindest einen Kopfbefestigungseinheit (20) fixierbar ausgeführt ist.

3. Schutzvorrichtung nach einem der vorhergehenden Ansprüche,
**dadurch gekennzeichnet, dass**
mittels der zumindest einen Einstelleinheit (24) eine direkte Distanz (d₁, d₂) zwischen den Augen (26) des Benutzers und dem optischen Blendschutzfilter (12) einstellbar ausgebildet ist.

4. Schutzvorrichtung nach einem der vorhergehenden Ansprüche,
**dadurch gekennzeichnet, dass**
mittels der zumindest einen Einstelleinheit (24) in einer Betriebsstellung eine Höhe (h₁, h₂) und/oder ein Winkel (α₁, α₂) der zumindest einen Schildeinheit (18) relativ zu der zumindest einen Kopfbefestigungseinheit (20) einstellbar ausgebildet ist.

5. Schutzvorrichtung nach einem der vorhergehenden Ansprüche,
**dadurch gekennzeichnet, dass**
die zumindest eine Schildeinheit (18) zumindest einen Gesichtsanschlag (28) aufweist, welcher in einer Endposition der Schildeinheit (18) dazu vorgesehen ist ein Gesicht des Benutzers zu kontaktieren.

6. Schutzvorrichtung nach einem der vorhergehenden Ansprüche,
**dadurch gekennzeichnet, dass**
der zumindest eine Gesichtsanschlag (28) von einer Nasenauflage gebildet ist, welche in zumindest einer Betriebsstellung zu einer Kontaktierung einer Nase (30) eines Benutzers vorgesehen ist.

7. Schutzvorrichtung nach Anspruch 6,
**dadurch gekennzeichnet, dass**
der zumindest eine Gesichtsanschlag (28) der zumindest einen Schildeinheit (18) zu einer Positionierung der zumindest einen Schildeinheit (18) relativ zu einem Gesicht eines Benutzers vorgesehen ist.

8. Schutzvorrichtung nach einem der vorhergehenden Ansprüche,
**dadurch gekennzeichnet, dass**
die Kopfbefestigungseinheit (20) in einem regulär getragenen Zustand eine Kopfachse (32) definiert, gegenüber welcher eine Haupterstreckungsebene (34) des optischen Blendschutzfilters (12) in zumindest einer Betriebsstellung wesentlich abgewinkelt ist.

9. Schutzvorrichtung nach einem der vorhergehenden Ansprüche,
**dadurch gekennzeichnet, dass**
die zumindest eine Kopfbefestigungseinheit (20) von einem Kopfband gebildet ist.

10. Schutzvorrichtung nach einem der vorhergehenden Ansprüche,
**dadurch gekennzeichnet, dass**
die zumindest eine Schildeinheit (18) aus einem zumindest im Wesentlichen formfesten Material besteht.

## Claims

1. Protection device, in particular glare protection device, with at least one optical glare protection filter (12) comprising a nose cut-out (14), with at least one sensor unit (16) that is configured to capture a work state and, depending thereon, to control a permeability of the glare protection filter (12), with at least one shield unit (18), in which the at least one glare protection filter (12) is fixedly accommodated, and with at least one head-fastening unit (20) for a fastening to a user's head (22), **characterised by** at least one adjusting unit (24), by which a position of the at least one shield unit (18) is movable relative to the at least one head-fastening unit (20) in a defined manner, wherein a position of the at least one shield unit (18) is fixable in at least two operative adjustments relative to the at least one head-fastening unit (20) by means of the at least one adjusting unit (24).

2. Protection device according to claim 1, **characterised in that** a position of the at least one shield unit (18) is fixable in at least two adjustments relative to the at least one head-fastening unit (20) by the at least one adjusting unit (24).

3. Protection device according to one of the preceding claims, **characterised in that** a direct distance (d₁, d₂) between the user's eyes (26) and the optical glare protection filter (12) is implemented to be adjustable by the at least one adjusting unit (24).

4. Protection device according to one of the preceding claims, **characterised in that** a height (h₁, h₂) and/or an angle (α₁, α₂) of the at least one shield unit (18) is implemented to be adjustable relative to the at least one head-fastening unit (20) in at least one operative adjustment by means of the at least one adjusting unit (24).

5. Protection device according to one of the preceding claims, **characterised in that** the at least one shield unit (18) comprises at least one face abutment (28), which is configured to contact a user's face in an end position of the shield unit (18).

6. Protection device according to one of the preceding claims, **characterised in that** the at least one face abutment (28) is embodied by a nosepiece, which is in at least one operative adjustment configured for contacting a user's nose (30).

7. Protection device according to claim 6, **characterised in that** the at least one face abutment (28) of the at least one shield unit (18) is configured for a positioning of the at least one shield unit (18) relative to a user's face.

8. Protection device according to one of the preceding claims, **characterised in that** the head-fastening unit (20) defines, in a regularly worn state, a head axis (32), relative to which a main extension plane (34) of the optical glare protection filter (12) is substantially angled in at least one operative adjustment.

9. Protection device according to one of the preceding claims, **characterised in that** the at least one head-fastening unit (20) is embodied as a head band.

10. Protection device according to one of the preceding claims, **characterised in that** the at least one shield unit (18) is made of an at least substantially dimensionally stable material.

## Revendications

1. Dispositif de protection, notamment dispositif anti-éblouissement, avec au moins un filtre optique anti-éblouissement (12) ayant une encoche-nez (14), avec au moins une unité capteur (16) prévue pour un captage d'un état opératif et, dépendant dudit état opératif, d'une commande de perméabilité du filtre anti-éblouissement (12), avec au moins une unité d'écran (18), dans laquelle l'au moins un filtre anti-éblouissement (12) est fixement agencé, et avec au moins une unité fixateur de tête (20) pour une fixation à une tête (22) d'un usager,
**caractérisé par** au moins une unité d'ajustement (24), par le biais de laquelle une position de l'au moins une unité d'écran (18) est mouvable de manière définie par rapport à l'au moins une unité fixateur de tête (20),
où une position de l'au moins une unité d'écran (18) est réalisée à être fixable par rapport à l'au moins une unité fixateur de tête (20) en au moins deux ajustements opératifs par le biais de l'au moins une unité d'ajustement (24).

2. Dispositif de protection selon la revendication 1,
**caractérisé en ce qu'une** position de l'au moins une unité d'écran (18) est réalisée à être fixable en au moins deux ajustements par rapport à l'au moins une unité fixateur de tête (20) par l'au moins une unité d'ajustement (24).

3. Dispositif de protection selon l'une quelconque des revendications précédentes,
**caractérisé en ce qu'une** distance directe (d₁, d₂) entre les yeux (26) de l'usager et le filtre optique anti-éblouissement (12) est réalisée à être ajustable par l'au moins une unité d'ajustement (24).

4. Dispositif de protection selon l'une quelconque des revendications précédentes,
**caractérisé en ce que,** dans un ajustement opératif, une hauteur (h₁, h₂) et/ou un angle (α₁, α₂) de l'au moins une unité d'écran (18) est/sont réalisée/é/s à être ajustable/s par rapport à l'au moins une unité fixateur de tête (20) par le biais de l'au moins une unité d'ajustement (24).

5. Dispositif de protection selon l'une quelconque des revendications précédentes,
**caractérisé en ce que** l'au moins une unité d'écran (18) comporte au moins un aboutement facial (28) prévu à contacter le visage de l'usager dans un positionnement final de l'unité d'écran (18).

6. Dispositif de protection selon l'une quelconque des revendications précédentes,
**caractérisé en ce que** l'au moins un aboutement facial (28) est réalisé par une nez-selle, qui est prévue à contacter le nez (30) d'un usager dans au moins un ajustement opératif.

7. Dispositif de protection selon la revendication 6,
**caractérisé en ce que** l'au moins un aboutement facial (28) de l'au moins une unité d'écran (189 est prévu pour un positionnement de l'au moins une unité d'écran (18) par rapport au visage d'un usager.

8. Dispositif de protection selon l'une quelconque des revendications précédentes,
**caractérisé en ce que** l'unité fixateur de tête (18), en état d'être régulièrement portée, définit un axe de tête (32), par rapport auquel un plan principal d'extension (34) du filtre optique anti-éblouissement (12) est sensiblement anglé dans au moins un ajustement opératif.

9. Dispositif de protection selon l'une quelconque des revendications précédentes,
**caractérisé en ce que** l'au moins une unité fixateur de tête (20) est réalisée comme bandeau-tête.

10. Dispositif de protection selon l'une quelconque des revendications précédentes,
**caractérisé en ce que** l'au moins une unité d'écran (18) est composée d'un matériau au moins sensiblement indéformable.
